(19) Europäisches Patentamt
European Patent Office
Office européen des brevets

(11) **EP 4 306 947 A1**

(12) **EUROPEAN PATENT APPLICATION**

(43) Date of publication:
**17.01.2024 Bulletin 2024/03**

(21) Application number: **22184953.2**

(22) Date of filing: **14.07.2022**

(51) International Patent Classification (IPC):
**G01N 27/327** (2006.01)   **G01N 33/543** (2006.01)
**G01N 33/78** (2006.01)

(52) Cooperative Patent Classification (CPC):
**G01N 27/3275; G01N 33/5438; G01N 33/78**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **HEI Therapeutics ApS**
**2200 København N (DK)**

(72) Inventors:
• **SVENDSEN, Winnie Edith**
**2200 København N (DK)**
• **ANDRESEN, Line**
**2200 København N (DK)**
• **LENDVAI, Ágnes Kovács**
**2200 København N (DK)**

(74) Representative: **Zacco Denmark A/S**
**Arne Jacobsens Allé 15**
**2300 Copenhagen S (DK)**

(54) **BIOSENSOR FOR DETECTION OF TSH LEVELS**

(57) The present disclosure relates an electrochemical biosensor system for measuring concentrations of an analyte (thyroid-stimulating hormone, TSH) in a fluid, the system comprising an electrochemical sensor having a working electrode having a binding agent attached to its surface (peptide comprising binding motif for TSH having sequences derived from or similar to thyroid-stimulating hormone receptor (TSHR)). The present disclosure further relates to uses of such biosensor, and methods of measuring with said biosensor in a sample to be analysed.

Figure 1

## Description

## Technical Field

The invention relates to an electrochemical biosensor system for measuring concentrations of an analyte in a fluid, the system comprising an electrochemical sensor having a working electrode having a binding agent attached to its surface. The invention further relates to uses of such biosensor, and methods of measuring with said biosensor in a sample to be analysed.

## Background art

Hypothyroidism (thyroid hormone deficiency) is prevalent in 5%-10% of the adult population. Worldwide, 200 million people suffer from hypothyroidism, with four million patients being newly diagnosed every year (800,000 in the EU and US). Hypothyroidism is a chronic illness and requires lifelong treatment through thyroid hormone replacement.

Current management of hypothyroidism relies on repeated blood testing for measuring the levels of the thyroid-stimulating hormone (TSH) in the blood. Analysis of TSH level is considered the frontline test for thyroid function assessment.

However, one of the major obstacles when treating hypothyroidism is the adjustment and fine tuning of the correct drug dosage needed to treat the patient. This is challenging as a new blood test is required every time the dosage is adjusted, which is very time-consuming and costly. This is of particular concern for newly diagnosed patients, for whom it takes an average of 12 months and 10 doctor visits to achieve the right treatment to reach stable TSH levels.

Moreover, dysregulated hormone substitution occurs at some point in time for around 50% of chronic patients despite lifelong treatment. The duration and extent of dysregulated TSH levels correlate with morbidity and mortality and may have an explicit impact on patient prognosis. Currently, patients with presumably stable TSH have yearly follow-ups, which results in a lack of clinical information between visits and possible undetectable dysregulations.

Besides reducing Quality of Life (QoL), inadequate treatment for hypothyroidism increases the risk of cardiac complications, dementia, mental health issues, and cancer, which emphasizes the need for tools to improve clinical management.

Further, hypothyroidism management results in a significant economic burden for patients and healthcare systems. In Denmark alone, millions TSH lab tests are performed every year costing more than millions of euro to the government. In the US, given the prevalence of hypothyroidism, there is also a substantial economic burden reaching billions of dollars annually. There is therefore a need to provide a better solution to monitor patients having hypothyroidism, which overcomes the above described problems with the present solutions.

## Summary

The present invention solves the above problems by providing a monitoring solution, which can serve as a point-of-care or home monitoring solution, which enables personalized treatment for hypothyroidism. Hence, disclosed herein in a first aspect is an electrochemical biosensor system for measuring concentrations of an analyte in a fluid, the electrochemical biosensor system comprises an electrochemical sensor, the electrochemical sensor comprising an electrode system comprising a working electrode, a counter electrode, and a reference electrode; wherein the working electrode comprises a first surface having a predefined electron transfer property, the first surface being in contact with the fluid when the electrochemical biosensor system is in use; wherein a binding agent is attached to the first surface, the binding agent being configured for specifically binding to the analyte to form a binding agent-analyte complex, wherein formation of the binding agent-analyte complex alters the predefined electron transfer property of the first surface of the working electrode, thereby providing a change in an electrochemical response at the first surface of the working electrode proportional to the number of binding agent-analyte complexes formed; and wherein the analyte is thyroid-stimulating hormone (TSH) and wherein the binding agent is one or more peptides comprising a binding motif for the analyte having sequences deriving from or similar to thyroid-stimulating hormone receptor (TSHR).

The present invention hereby discloses a unique medical device, which is a peptide-based electrochemical biosensor for the measurement of TSH. The device can e.g. be made with disposable test strips that provides a health monitoring solution to the patient or to the healthcare system. This may be used to obtain a better medical insight into the patients TSH levels over time and hereby provide a better opportunity to fine tune the drug dosage, even over time at any point during treatment.

Further, the disclosed invention may also help assisting in providing information on medication and lifestyle helps to manage life with chronic thyroid disease. In one or more embodiments, the device may be able to detect TSH in a drop of plasma/serum (e.g. $15\mu l$) in a clinical measurement range.

Peptides are versatile tools for the design and development of biosensors due to their costefficiency, high stability, and selectivity to their targets. The present invention utilizes peptides based on the interaction of TSH and its receptor, thyroid stimulating hormone receptor (TSHR).

By utilizing peptides for TSH detection the cost may e.g. be reduce per tests by a factor of 20 to 30 times, compared to current antibody-based technologies used within the area today. Further, the electrochemical sensors ensure usability for point-of-care analysis making it easier for patients to do home testing and always evaluate their current TSH levels, hereby also minimizing the risk of dysregulated hormone substitution to occur, or treating it as early as possible, if occurring.

Further, the system may provide an "all in one" user-friendly blood sample handling system for TSH monitoring, hereby making it even easier for patients to do home testing.

The system as disclosed herein provides a clever, new, and smart way to monitor thyroid disease and adjust medication more precisely based on up-to-date patient data analysis.

The biosensor system is configured to detect thyroid-stimulating hormone (TSH) that includes peptide(s) immobilized on a sensing surface, such as a gold electrode. The immobilized peptide(s) comprise a binding motif for the target (TSH). The sensing component may be based on an electrical impedance that changes in response to the binding of the immobilized peptide to the target. The immobilized peptide specifically binds to the target (TSH). The biosensor system hereby provides a readout that is responsive to changes of the sensing component, such as changes in electrical impedance of the sensing component.

The present invention further discloses a composition that comprises peptides derived from Thyroid Stimulating Hormone Receptor (TSHR). The composition or peptides may be useful in the detection of TSH, which is useful in the monitoring of treatment and/or prevention of adverse effects of thyroid diseases such as hypothyroidism.

Disclosed herein in a second aspect is a use of electrochemical biosensor system according to the first aspect for measuring a concentration of an analyte in a fluid.

Disclosed herein in a third aspect is a use of electrochemical biosensor system according to the first aspect for monitoring treatment of thyroid diseases, such as hypothyroidism.

Disclosed herein in a fourth aspect is a use of electrochemical biosensor system according to the first aspect for preventing adverse effects of thyroid diseases, such as hypothyroidism.

Disclosed herein in a fifth aspect is a method of measuring a concentration of an analyte in a fluid, the method comprising:

- providing an electrochemical biosensor system according to the first aspect;
- providing a fluid sample;
- contacting the working electrode with the fluid sample under conditions that allow the formation of the binding agent-analyte complexes for a period of time sufficient to allow the formation of the binding agent-analyte complexes.

Disclosed herein in a sixth aspect is a method for monitoring treatment of thyroid diseases, such as hypothyroidism, the method comprising:

- providing an electrochemical biosensor system according to the first aspect;
- providing a fluid sample;
- contacting the working electrode with the fluid sample under conditions that allow the formation of the binding agent-analyte complexes for a period of time sufficient to allow the formation of the binding agent-analyte complexes.

Disclosed herein in a seventh aspect is a method for preventing adverse effects of thyroid diseases, such as hypothyroidism, the method comprising:

- providing an electrochemical biosensor system according to the first aspect;
- providing a fluid sample;
- contacting the working electrode with the fluid sample under conditions that allow the formation of the binding agent-analyte complexes for a period of time sufficient to allow the formation of the binding agent-analyte complexes.

Effects and features of the second-seventh aspects are to a large extent analogous to those described above and below in connection with the first aspect. Embodiments mentioned in relation to any aspect are largely compatible with the other aspects.

**Brief description of the drawings**

**Figure 1** is a schematic representation of applied surface chemistry according to an embodiment of the invention.

**Figure 2** shows signals of plasma samples spiked with different TSH concentrations.

**Figure 3** shows signal correlation between natural plasma vs TSH spiked plasma samples.

**Figure 4** shows plasma samples with different original TSH level and their EIS signal (A); with corresponding calibration curve (B), and Calculated LOD (C).

**Figure 5** shows EIS results of TSH at different concentrations in accordance with example 3.

**Figure 6** shows the results of a precision and accuracy test.

**Figure** 7 shows a specificity test of a specific peptide according to an embodiment of the invention.

**Detailed description**

The present disclosure will become apparent from the detailed description given below. The detailed description and specific examples disclose preferred embodiments of the disclosure by way of illustration only. Those skilled in the art understand from guidance in the detailed description that changes and modifications may be made within the scope of the disclosure.

The description herein of any aspect or embodiment using terms such as "comprising", "having," "including," or "containing" with reference to an element or elements is intended to provide support for a similar aspect or embodiment of the invention that "consists of", "consists essentially of", or "substantially comprises" that particular element or elements, unless otherwise stated or clearly contradicted by context, e.g. a composition described herein as comprising a particular element should be understood as also describing a composition consisting of that element, unless otherwise stated or clearly contradicted by context. It will be further understood that the terms "comprises," "comprising," "includes" and/or "including," when used in this specification, specify the presence of stated features, integers, steps, operations, elements, and/or components, but do not preclude the presence or addition of one or more other features, integers, steps, operations, elements, components, and/or groups thereof.

As used herein, the singular forms "a," "an," and "the" are intended to include the plural forms, including "at least one," unless the content clearly indicates otherwise. "At least one" is not to be construed as limiting "a" or "an."

The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

This invention includes all modifications and equivalents of the subject matter recited in the claims and/or aspects appended hereto as permitted by applicable law.

Unless otherwise defined, all terms used herein (including technical and scientific terms) have the same meaning as commonly understood by those skilled in the art to which this invention pertains. It will be further understood that terms, such as those defined in commonly used dictionaries, should be interpreted as having a meaning that is consistent with their meaning in the context of the relevant art and will not be interpreted in an idealized or overly formal sense unless expressly so defined in the present specification.

When describing the below embodiments, the present invention envisages all possible combinations and permutations of the below described embodiments with the above disclosed aspects.

As disclosed herein and above, the present invention relates to an electrochemical biosensor system for measuring concentrations of an analyte in a fluid, the system comprising an electrochemical sensor having a working electrode having a binding agent attached to its surface. The invention further relates to uses of such biosensor, and methods of measuring with said biosensor in a sample to be analysed.

In contrast to conventional biosensors where antibodies are utilized, the simple electrochemical biosensor system employs a peptide sequence as biomimetic receptors for target recognition. Unlike some of their biological counterparts, peptides have high robustness and stability under a wide range of conditions, the recognition sites for the specific detection can be designed, and it is low in production cost.

An electrochemical biosensor is a device that gives information about a composition by coupling a chemically selective layer/or a recognition element, to an electrochemical transducer. In this way, the chemical energy of the selective interaction between the target species and the sensor is transduced into an analytically useful signal.

In one or more embodiments, the first surface of the working electrode is made of a noble metal.

A working electrode is an electrode in an electrochemical system on which the reaction of interest is occurring.

The working electrode is often used in conjunction with an auxiliary electrode, and a reference electrode in a three electrode system. Depending on whether the reaction on the electrode is a reduction or an oxidation, the working electrode is called cathodic or anodic, respectively. Common working electrodes can consist of materials ranging from inert metals such as gold, silver or platinum, to inert carbon such as glassy carbon, boron doped diamond or pyrolytic carbon, and mercury drop and film electrodes.

In one or more embodiments, the noble metal is copper, silver, platinum, gold, bismuth, palladium, osmium, iridium, ruthenium, rhodium, or combinations thereof.

In one or more embodiments, the noble metal is gold.

In one or more embodiments, the working electrode comprise a material selected from the group of platinum, gold, titanium, silver, copper, carbon, carbon nanotube, graphite, or combinations thereof.

In one or more embodiments, the working electrode is a gold electrode.

In one or more embodiments, the electrochemical biosensor system is for point-of-care testing.

In one or more embodiments, the working electrode has an electrical impedance, wherein the electrical impedance changes in response to the formation of the binding agent-analyte complex.

In one or more embodiments, the binding agent is immobilized on the first surface of the working electrode.

In one or more embodiments, the electrochemical biosensor system further comprises a computing unit cable of receiving a first input from the electrode system, the first input corresponding to the change in the electrochemical response at the first surface of the working electrode proportional to the number of binding agent-analyte complexes formed, and wherein the computing unit configured for transmitting data to an end user based on the received first input.

In one or more embodiments, the electrochemical biosensor system is for monitoring treatment of thyroid diseases, such as hypothyroidism.

In one or more embodiments, the electrochemical biosensor system is for preventing adverse effects of thyroid diseases, such as hypothyroidism.

In one or more embodiments, the liquid is selected from a buffer solution, deionized water, an organic solution, a biological solution, liquid food products, or combinations thereof.

In one or more embodiments, the biological solution is selected from sweat, tears, blood, urine, blood plasma, blood serum, or combinations thereof.

In one or more embodiments, the liquid is in a volume of 50 microliters ($\mu$L) or less, such as 40 microliters or less, such as 30 microliters or less, such as 25 microliters or less, such as less than 20 microliters or less, or such as 15 microliters or less.

In one or more embodiments, the thyroid-stimulating hormone receptor (TSHR) is selected from SEQ ID 1-36 as shown below.

SEQ ID 1 (meoY)DGH(Yno2)PYT-amine
SEQ ID 2 (meoY)DFE(Yno2)PYT-amine
SEQ ID 3 (meoY)DGH(Yno2)PYT
SEQ ID 4 (meoY)DGE(Yno2)PYT-amine
SEQ ID 5 QAFDSH(Ys)DYTI
SEQ ID 6 FNGQ(Yno2)PFT-amine
SEQ ID 7 Ace-FDSH(Yno2)DYTI
SEQ ID 8 FSGH(Yno2)DYT-amine
SEQ ID 9 FSGH(Yno2)DYT
SEQ ID 10 (meoY)DFE(Yno2)PYT
SEQ ID 11 FSGH(Yno2)PYT
SEQ ID 12 YSGH(Yno2)PYT-amine
SEQ ID 13 (meoY)DGE(Yno2)PYT
SEQ ID 14 (meoY)DGE(Yno2)DYT
SEQ ID 15 FSH(Yno2)DYT
SEQ ID 16 QAFDSH(Yno2)DYTI
SEQ ID 17 FSH(Yno2)DYTI
SEQ ID 18 FDGH(Yno2)DYT
SEQ ID 19 FDSHY
SEQ ID 20 AFDSHYDY
SEQ ID 21 FDSHYDY
SEQ ID 22 AFDSHYDY
SEQ ID 23 FDSHYDYTI
SEQ ID 24 AFDSHYDYTI
SEQ ID 25 AFDSHYDYTIC

SEQ ID 26 LQAFDSHYDYTI
SEQ ID 27 LQAFDSHYDYTIC
SEQ ID 28 TLQAFDSHYDYTIC
SEQ ID 29 EETLQAFDSHYDYTI
SEQ ID 30 EETLQAFDSHYDYTIC
SEQ ID 31 EETLQAFDSHYDYTICG
SEQ ID 32 PQEETLQAFDSHYDYTIC
SEQ ID 33 NPQEETLQAFDSHYDYTIC
SEQ ID 34 KNPQEETLQAFDSHYDYTIC
SEQ ID 35 CRRAFDSHYDYTI
SEQ ID 36 QAFDSHYDYTI

In SEQ ID 1-36, A is alanine, C is cysteine, D is aspartic acid, E is glutamic acid, F is phenylalanine, G is glycine, H is histidine, I is isoleucine, K is lysine, L is leucine, N is asparagine, P is proline, Q is glutamine, R is R-arginine, S is serine, T is threonine, Y is tyrosine, Ys is sulphated tyrosine, Yno2 is nitro tyrosine, meoY is O-methylated tyrosine, Ace is acetylation of the N terminus, and Amine is amination of the C terminus.

In one or more embodiments, the sequences deriving from or similar to thyroid-stimulating hormone receptor (TSHR) comprises at least 7 consecutive amino acids in the binding motif having the sequence $X_1X_2X_3X_4X_5X_6X_7$, wherein $X_1$ is selected from a phenylalanine, a tyrosine, or an O-methylated tyrosine; $X_2$ is selected from an aspartic acid, a serine, or an asparagine; $X_3$ is selected from a serine, a glycine or a phenylalanine; $X_4$ is selected from a histidine, a glutamine, or a glutamic acid; $X_5$ is selected from a tyrosine amino acid or derivatives hereof, such as a sulphated tyrosine, an O-methylated tyrosine, or a nitro tyrosine; $X_6$ is selected from an aspartic acid or a proline; $X_7$ is selected from a tyrosine or a phenylalanine, and wherein said peptide has a length of at least 8 amino acids, such as at least 10 amino acids, or such as at least 12 amino acids.

In one or more embodiments, $X_5$ is a sulfated tyrosine amino acid derivative.

SEQ ID 36 is the original sequence from human TSHR. Modification to this seqeucence can in some instances have the following effect, depending on the sequence and the modification:

- By adding R (R-arginine) the sequence may show an improve isoelectric point and solubility.
- By adding C (cysteine), C and a spacer, such as e.g. a PEG4 spacer, or a thiolated spacer, such as e.g. a thiol-PEG4 spacer, it could further help to ensure the direct attachment of the peptide to the gold electrode surface.
- By modification of Y to Ys increases the binding affinity.
- By modifictaion of Ys to Yno2 increase interaction.
- Q and A can be removed from the original sequence as they may not affect the overall binding affinity.
- Acetylation of N-terminal may increase stability.
- Modification with Ace (acetylation of N terminus) may improve binding.
- Removal of I may increase ligand efficiency.
- Modification of S to G may improve binding.
- Modification of D to S may improve the electrostatic contribution.
- Addition of amine (amination to C terminus) may increase solubility.
- Modification of D to P may increase interaction stability.
- Modification of F to Y and addition of amine (amination to C terminus) may increase isoelectric point.
- Modification of S to N, H to Q and Y to F, in addition of amine (amination to C terminus) may improve solubility.
- Modification of F to meoY, S to F, and H to E, in addition to amine (amination to C terminus) may improve solubility.
- Modification of S to G may improve solubility.
- Modification of H to E may improve solubility.
- Modification of P to D may improve solubility.
- Modification of G to F and D to P may improve solubility.

In one or more embodiments, the binding agent further comprises a cysteine amino acid in one end covalently bound to the first surface of the working electrode.

In one or more embodiments, the binding agent further comprises one or more arginine and/or lysine amino acids.

In one or more embodiments, the binding agent is attached to the first surface of the working electrode via a linker molecule attached between the first surface and the binding agent.

In one or more embodiments, the linker is a PEG linker.

In one or more embodiments, the binding agent is covalently bound to the first surface of the working electrode.

In one or more embodiments, the electrochemical biosensor system further comprises a potentiostat and a microprocessor, wherein the working electrode, the counter electrode, and the reference electrode are connected to the potentiostat, and wherein the potentiostat is connected to the microprocessor.

The analog part of the system consists of a potentiostat including the working electrode, counter electrode, and reference electrode. The potentiostat sets the voltage between the working electrode (WE) and the reference electrode (RE) in the sample under test, by providing the necessary current through the counter electrode (CE). The usage of the potentiostat is to apply a voltage and to record the current response that is characteristic of the sample being tested.

A microcontroller may be used to generate the voltage waveform for the potentiostat to perform the electrochemical measurements and acquires the data. By using a microcontroller low cost, low power consumption, and sufficient computation capability is achieved.

The microprocessor is used to generate required voltage signals and communicate with an end user. Using microprocessor for both data acquiring and transmitting makes the system portable, minimizes the size of the device, and reduces the power consumption.

In one or more embodiments, the microprocessor is a digital microprocessor.

In one or more embodiments, the microprocessor is adapted for receiving data from the electrochemical sensor and transmitting data to an end user.

In one or more embodiments, the working electrode has a form selected from the group of a mesh, a wire, a flag, a sheet, a bar, a three-dimensional sponge, a three-dimensional microneedle array, or combinations thereof.

In one or more embodiments, the counter electrode comprises a material selected from the group of platinum, gold, titanium, silver, copper, carbon, carbon nanotube, graphite, or combinations hereof.

A counter electrode, also known as an auxiliary electrode, is an electrode used in a threeelectrode electrochemical cell for voltammetric analysis or other reactions in which an electric current is expected to flow. The counter electrode is distinct from the reference electrode, which establishes the electrical potential against which other potentials may be measured, and the working electrode, at which the cell reaction takes place.

In one or more embodiments, the counter electrode has a form selected from the group of a mesh, a wire, a flag, a sheet, a bar, a three-dimensional sponge, a three-dimensional microneedle array, or combinations thereof.

In one or more embodiments, the reference electrode comprises a material selected from the group of platinum, gold, titanium, silver, copper, carbon, carbon nanotube, graphite, or combinations thereof.

A reference electrode is an electrode which has a stable and well-known electrode potential. The high stability of the electrode potential is usually reached by employing a redox system with constant, buffered or saturated, concentrations of each participant of a redox reaction.

In one or more embodiments, the reference electrode has a form selected from the group of a mesh, a wire, a flag, a sheet, a bar, a three-dimensional sponge, a three-dimensional microneedle array, or combinations thereof.

In one or more embodiments, the reference electrode is selected from the group of a silver/silver chloride electrode, a platinized platinum electrode, or a calomel electrode.

In one or more embodiments, the electrochemical biosensor system comprises two parts detachably connected, the two parts including:

a disposable part comprising at least the electrode system comprising the working electrode, the counter electrode, and the reference electrode; and a reusable part comprising a potentiostat and a microprocessor;

wherein the working electrode, the counter electrode, and the reference electrode are connected to the potentiostat, and wherein the potentiostat is connected to the microprocessor.

The design of disposable and reusable parts minimizes the cost of each test. The user only needs to change the disposable chip for a new test.

By detachably connected is meant that the two parts can be separated/disconnected from each other without breaking parts of the electrochemical biosensor system.

When an element is referred to as being "connected" to another element, the element is "directly connected" to the other element, or "electrically connected" to the other element with one or more intervening elements interposed therebetween.

In one or more embodiments, the electrochemical biosensor system further comprises a sample handling unit.

In one or more embodiments, the sample handling unit is in the disposable part.

In one or more embodiments, the sample handling unit comprises an integrated blood to plasma separation unit.

In one or more embodiments, the electrochemical biosensor system further comprises a flow control.

In one or more embodiments, the flow control comprises an automatic reagent release system configured to release plasma and one or more reagents to the electrode system.

In one or more embodiments, at least one of the one or more reagents are ferrocyanide.

In one or more embodiments, the electrochemical biosensor system further comprises a waste collection unit.

In one or more embodiments, the electrochemical biosensor system further comprises an additional sensor system.

In one or more embodiments, the additional sensor system is configured to ensure a predefined quality of a readout provided by the electrode system.

In one or more embodiments, the system is a sensor strip-based system comprising a disposable part in the form of a sensor strip and reusable part, wherein the sensor strip comprises a sample handling unit that has an integrated blood to plasma separation unit, a flow control with automatic and timely release of plasma and reagent (such as ferrocyanide), and a waste collection unit.

In one or more embodiments, the system comprising a disposable part and reusable part, wherein the reusable part comprises an integrated miniature potentiostat and a read-out unit configured to provide a readout to an end user. It is the reusable part, which ensures the electrochemical measurements.

In one or more embodiments, the fluid sample is whole blood sample from a human subject.

In one or more embodiments, the whole blood has been collected from a finger of the human subject.

In one or more embodiments, the whole blood sample is separated to at least two subsamples using a blood separation unit within the electrochemical biosensor system, wherein one of these subsamples is a plasma sample.

In one or more embodiments, the method further comprises the step of providing an electrochemical impedance spectroscopy measurement and providing said measurement to an end user.

In one or more embodiments, an analyte measurement is done by providing a whole blood sample, such as a whole blood sample from a finger of a subject. This sample, when added to the system will reach the blood separation unit, thus plasma will be separated. The plasma sample passes on the microfluidic system to the sensor unit, where after a short incubation of the sample on the sensor surface, the probe reagent solution is released, and electrochemical impedance spectroscopy measurement is carried out. The measurements are then directly or indirectly provided to the end user.

In one or more embodiments, the working electrode of the electrochemical biosensor system can be produced by:

- providing a working electrode,
- cleaning of the electrode,
- functionalization of the working electrode with a peptide sequence as disclosed herein,
- removing redundant peptides from the working electrode,
- blocking the surface of the working electrode with a suitable blocking agent, and
- removing redundant blocking agent from the working electrode,

In one or more embodiments, a sample can be tested by:

- providing a sample to be tested, preferably a liquid sample, such as a whole blood sample,
- optionally separating the sample into at least two fractions, such as a plasma serum sample,
- optionally adding a buffer, such as a standard buffer to the sample
- incubating the sample with the working electrode in the system,
- removing redundant sample from the working electrode,
- adding a probe reagent solution, such as a (ferri/ferrocyanide solution), and
- measuring electrochemical impedance (EIS).

EIS is a multifrequency AC electrochemical measurement technique. It measures the electrical resistance (impedance) of the metal/solution interface over a wide range of frequencies (from 1 mHz to 10 kHz). Electrochemical impedance measurement is by applying an AC potential to an electrochemical cell and then measuring the current through the cell. The applied AC excitation takes on the form of sinusoidal waves which are time and amplitude dependent based on the applied frequency. The binding event between the peptide and analyte is signalled by an increase in impedance, more specifically an increase in charge transfer resistance due to the passivation of the conductive electrode surface. This offers information into the presence and amount of the target analyte.

The invention will hereafter be described by way of the following non-limiting items.

1. An electrochemical biosensor system for measuring concentrations of an analyte in a fluid, the electrochemical biosensor system comprises an electrochemical sensor, the electrochemical sensor comprising an electrode system comprising a working electrode, a counter electrode, and a reference electrode;

wherein the working electrode comprises a first surface having a predefined electron transfer property, the first surface being in contact with the fluid when the electrochemical biosensor system is in use;

wherein a binding agent is attached to the first surface, the binding agent being configured for specifically binding to the analyte to form a binding agent-analyte complex, wherein formation of the binding agent-analyte complex alters the predefined electron transfer property of the first surface of the working electrode, thereby providing a change in an electrochemical response at the first surface of the working electrode proportional to the number of binding agent-analyte complexes formed; and

wherein the analyte is thyroid-stimulating hormone (TSH) and wherein the binding agent is one or more peptides comprising a binding motif for the analyte having sequences deriving from or similar to thyroid-stimulating hormone receptor (TSHR).

2. The electrochemical biosensor system according to item 1, wherein the first surface of the working electrode is made of a noble metal.

3. The electrochemical biosensor system according to item 2, wherein the noble metal is copper, silver, platinum, gold, bismuth, palladium, osmium, iridium, ruthenium, rhodium, or combinations thereof.

4. The electrochemical biosensor system according to item 2, wherein the noble metal is gold.

5. The electrochemical biosensor system according to any of the preceding items, wherein the working electrode comprise a material selected from the group of platinum, gold, titanium, silver, copper, carbon, carbon nanotube, graphite, or combinations thereof.

6. The electrochemical biosensor system according to item 5, wherein the working electrode is a gold electrode.

7. The electrochemical biosensor system according to any of the preceding items, wherein the electrochemical biosensor system is for point-of-care testing.

8. The electrochemical biosensor system according to any of the preceding items, wherein the working electrode has an electrical impedance, wherein the electrical impedance changes in response to the formation of the binding agent-analyte complex.

9. The electrochemical biosensor system according to any of the preceding items, wherein the binding agent is immobilized on the first surface of the working electrode.

10. The electrochemical biosensor system according to any of the preceding items, wherein the electrochemical biosensor system further comprises a computing unit cable of receiving a first input from the electrode system, the first input corresponding to the change in the electrochemical response at the first surface of the working electrode proportional to the number of binding agent-analyte complexes formed, and wherein the computing unit configured for transmitting data to an end user based on the received first input.

11. The electrochemical biosensor system according to any of the preceding items, wherein the electrochemical biosensor system is for monitoring treatment of thyroid diseases, such as hypothyroidism.

12. The electrochemical biosensor system according to any of the preceding items, wherein the electrochemical biosensor system is for preventing adverse effects of thyroid diseases, such as hypothyroidism.

13. The electrochemical biosensor system according to any of the preceding items, wherein the liquid is selected from a buffer solution, deionized water, an organic solution, a biological solution, liquid food products, or combinations thereof.

14. The electrochemical biosensor system according to item 13, wherein the biological solution is selected from sweat, tears, blood, urine, blood plasma, blood serum, or combinations thereof.

15. The electrochemical biosensor system according to any of the preceding items, wherein the liquid is in a volume of 50 microliters ($\mu$L) or less, such as 40 microliters or less, such as 30 microliters or less, such as 25 microliters or less, such as less than 20 microliters or less, or such as 15 microliters or less.

16. The electrochemical biosensor system according to any of the preceding item, wherein the sequences deriving from or similar to thyroid-stimulating hormone receptor (TSHR) comprises at least 7 consecutive amino acids in the binding motif having the sequence $X_1X_2X_3X_4X_5X_6X_7$, wherein $X_1$ is selected from a phenylalanine, a tyrosine, or an O-methylated tyrosine; $X_2$ is selected from an aspartic acid, a serine, or an asparagine; $X_3$ is selected from a serine, a glycine or a phenylalanine; $X_4$ is selected from a histidine, a glutamine, or a glutamic acid; $X_5$ is selected from a tyrosine amino acid or derivatives hereof, such as a sulphated tyrosine, an O-methylated tyrosine, or a nitro

tyrosine; $X_6$ is selected from an aspartic acid or a proline; $X_7$ is selected from a tyrosine or a phenylalanine, and wherein said peptide has a length of at least 8 amino acids, such as at least 10 amino acids, or such as at least 12 amino acids.

17. The electrochemical biosensor system according to any of the preceding items, wherein the binding agent further comprises a cysteine amino acid in one end covalently bound to the first surface of the working electrode.

18. The electrochemical biosensor system according to any of the preceding items, wherein the binding agent further comprises one or more arginine and/or lysine amino acids.

19. The electrochemical biosensor system according to any of the preceding items, wherein the binding agent is attached to the first surface of the working electrode via a linker molecule attached between the first surface and the binding agent.

20. The electrochemical biosensor system according to item 19, wherein the linker is a PEG linker.

21. The electrochemical biosensor system according to any of the preceding items, wherein the binding agent is covalently bound to the first surface of the working electrode.

22. The electrochemical biosensor system according to item 16, wherein X is a sulfated tyrosine amino acid derivative.

23. The electrochemical biosensor system according to any of the preceding items, wherein the electrochemical biosensor system further comprises a potentiostat and a microprocessor, wherein the working electrode, the counter electrode, and the reference electrode are connected to the potentiostat, and wherein the potentiostat is connected to the microprocessor.

24. The electrochemical biosensor system according to item 23, wherein the microprocessor is a digital microprocessor.

25. The electrochemical biosensor system according to any of items 23-24, wherein the microprocessor is adapted for receiving data from the electrochemical sensor and transmitting data to an end user.

26. The electrochemical biosensor system according to any of the preceding items, wherein the working electrode has a form selected from the group of a mesh, a wire, a flag, a sheet, a bar, a three-dimensional sponge, a three-dimensional microneedle array, or combinations thereof.

27. The electrochemical biosensor system according to any of the preceding items, wherein the counter electrode comprises a material selected from the group of platinum, gold, titanium, silver, copper, carbon, carbon nanotube, graphite, or combinations hereof.

28. The electrochemical biosensor system according to any of the preceding items, wherein the counter electrode has a form selected from the group of a mesh, a wire, a flag, a sheet, a bar, a three-dimensional sponge, a three-dimensional microneedle array, or combinations thereof.

29. The electrochemical biosensor system according to any of the preceding items, wherein the reference electrode comprises a material selected from the group of platinum, gold, titanium, silver, copper, carbon, carbon nanotube, graphite, or combinations thereof.

30. The electrochemical biosensor system according to any of the preceding items, wherein the reference electrode has a form selected from the group of a mesh, a wire, a flag, a sheet, a bar, a three-dimensional sponge, a three-dimensional microneedle array, or combinations thereof.

31. The electrochemical biosensor system according to any of the preceding items, wherein the reference electrode is selected from the group of a silver/silver chloride electrode, a platinized platinum electrode, or a calomel electrode.

32. The electrochemical biosensor system according to any of the preceding items, wherein the electrochemical biosensor system comprises two parts detachably connected, the two parts including:

a disposable part comprising at least the electrode system comprising the working electrode, the counter electrode, and the reference electrode; and
a reusable part comprising a potentiostat and a microprocessor;

wherein the working electrode, the counter electrode, and the reference electrode are connected to the potentiostat, and wherein the potentiostat is connected to the microprocessor.

33. The electrochemical biosensor system according to any of the preceding items, wherein the electrochemical biosensor system further comprises a sample handling unit.

34. The electrochemical biosensor system according to item 33, wherein the sample handling unit comprises an integrated blood to plasma separation unit.

35. The electrochemical biosensor system according to item 33, wherein the sample handling unit is in the disposable part.

36. The electrochemical biosensor system according to any of the preceding items, wherein the electrochemical biosensor system further comprises a flow control.

37. The electrochemical biosensor system according to item 36, wherein the flow control comprises an automatic reagent release system configured to release plasma and one or more reagents to the electrode system.

38. The electrochemical biosensor system according to item 37, wherein at least one of the one or more reagents are ferrocyanide.

39. The electrochemical biosensor system according to any of the preceding items, wherein the electrochemical biosensor system further comprises a waste collection unit.

40. The electrochemical biosensor system according to any of the preceding items, wherein the electrochemical biosensor system further comprises an additional sensor system.

41. The electrochemical biosensor system according to item 40, wherein the additional sensor system is configured to ensure a predefined quality of a readout provided by the electrode system.

42. Use of electrochemical biosensor system according to any of the preceding items for measuring a concentration of an analyte in a fluid.

43. Use of electrochemical biosensor system according to any of items 1-41 for monitoring treatment of thyroid diseases, such as hypothyroidism.

44. Use of electrochemical biosensor system according to any of items 1-41 for preventing adverse effects of thyroid diseases, such as hypothyroidism.

45. A method of measuring a concentration of an analyte in a fluid, the method comprising:

a. providing an electrochemical biosensor system according to any of items 1-41;
b. providing a fluid sample;
c. contacting the working electrode with the fluid sample under conditions that allow the formation of the binding agent-analyte complexes for a period of time sufficient to allow the formation of the binding agent-analyte complexes.

46. A method for monitoring treatment of thyroid diseases, such as hypothyroidism, the method comprising:

a. providing an electrochemical biosensor system according to any of items 1-41;
b. providing a fluid sample;
c. contacting the working electrode with the fluid sample under conditions that allow the formation of the binding agent-analyte complexes for a period of time sufficient to allow the formation of the binding agent-analyte complexes.

47. A method for preventing adverse effects of thyroid diseases, such as hypothyroidism, the method comprising:

    a. providing an electrochemical biosensor system according to any of items 1-41;
    b. providing a fluid sample;
    c. contacting the working electrode with the fluid sample under conditions that allow the formation of the binding agent-analyte complexes for a period of time sufficient to allow the formation of the binding agent-analyte complexes.

48. The method according to any of items 45-47, wherein the analyte is thyroid-stimulating hormone (TSH).

49. The method according to any of items 45-48, wherein the fluid sample is whole blood sample from a human subject.

50. The method according to item 49, wherein the whole blood has been collected from a finger of the human subject.

51. The method according to any of items 49-50, wherein the whole blood sample is separated to at least two subsamples using a blood separation unit within the electrochemical biosensor system, wherein one of these subsamples is a plasma sample.

52. The method according to any of items 45-51, wherein the method further comprises the step of providing an electrochemical impedance spectroscopy measurement and providing said measurement to an end user.

The above objects, as well as additional objects, features and advantages of the present disclosure will be more fully appreciated by reference to the following illustrative and non-limiting detailed description of example embodiments of the present disclosure, when taken in conjunction with the accompanying drawings.

**Detailed description of the drawings**

The present disclosure will therefore now be described with reference to the accompanying drawings, in which preferred example embodiments of the disclosure are shown. The disclosure may, however, be embodied in other forms and should not be construed as limited to the herein disclosed embodiments. The disclosed embodiments are provided to fully convey the scope of the disclosure to the skilled person.

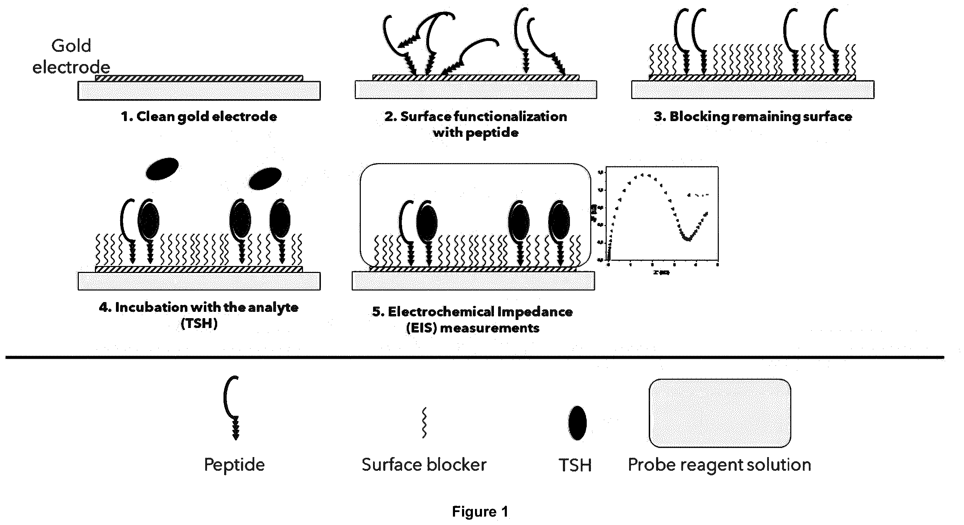

Figure 1 shows the working principle of the developed electrochemical method for the detection of TSH in complex biological sample. Step 1) Gold electrode is used as working electrode, where first all the possible contamination was removed to ensure a clean gold surface for attaching the biosensing element. Step 2) The biosensing element, here is a peptide, which specifically binds to TSH, is immobilized onto the electrode surface through the covalent binding between thiol group of cysteine (localized at the N-teminus of the peptide) and gold. Step 3) The electrode surface further treated with the surface blocking agent e.g. MCH to align the peptide based biosensing layer and block the remaining uncovered areas on the electrode surface. Step 4) The functionalized electrode surface was incubated with the analyte, here TSH, to capture the analyte by the biosensing element, here a TSH specific peptide. Step 5) For detecting the interaction between the peptide layer on the working electrode surface and TSH, a probe reagent, here ferri/ferrocyanide, was applied to perform electrochemical impedance measurement (EIS) and analyse the altered charge transfer resistance.

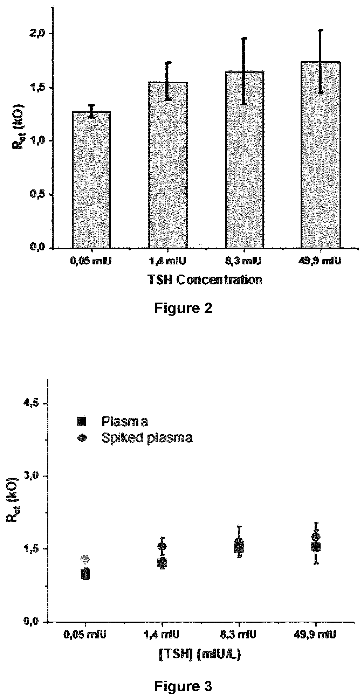

Figure 2 shows plasma samples (0.05 mIU/L), which were artificially spiked at corresponding TSH concentrations of the natural plasma samples in order to determine the correlation of known naturally occurring TSH vs the controlled laboratory scenario. A similar trend and correlation were observed between natural and artificially spiked plasma. This suggests that the system as disclosed herein is reproducible and can quantitatively measure TSH in complex media (for more details, see the examples).

Figure 3 shows a linear regression fit, which is applied in the range of 0.05-8.3 mIU/L in order to determine the biosensing performance of the biosensor system. Following the fitting results, it can be concluded that linearity is maintained at low TSH concentration in patient plasma samples and the observed limit of detection (LOD) is 0.05 mIU/L, while the calculated LOD is 0.84 mIU/L. Calculated LOD was obtained from 3xSD/(slope), where the SD is the standard deviation of the slope from the linear fitting. The calculated %RSD values at 0.05 mIU/L and 8.3 mIU/L TSH were 9.3% and 9.2%, respectively, showing a very good repeatability of the method.

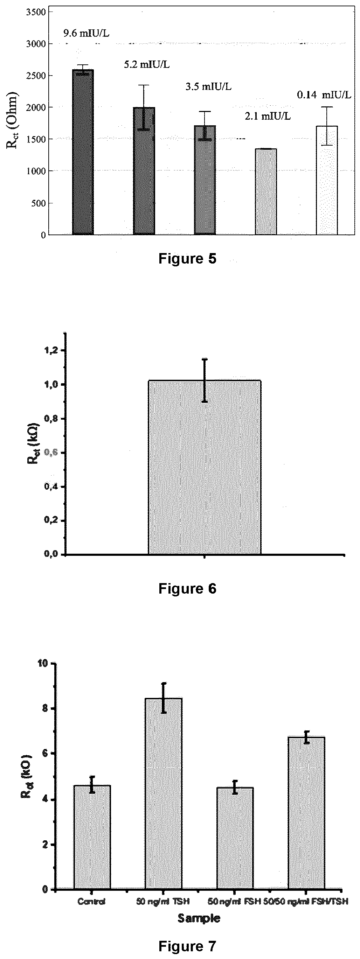

Figure 6 shows the repeatability of the method evaluated by taking twelve independent measurements in 5.0 mIU/L patient plasma sample. The calculated relative standard deviation (RSD) was found to be 12.2%. Similarly, the accuracy test was evaluated by taking twelve independent measurements in 6.5 mIU/L plasma sample and the calculated concentration using a calibration equation and was found to be 6.48 mIU/L, which is in agreement with the clinical analysis value.

Figure 7 shows a specificity test, in which an embodiment of the invention was tested towards specificity of thyroid stimulating hormone (TSH) versus follicle stimulating hormone (FSH).

The present invention is further illustrated by the following examples, which are not to be construed as limiting the scope of protection. The features disclosed in the foregoing description and in the following examples may, both separately or in any combination thereof, be material for realising the invention in diverse forms thereof.

**Examples**

Example 1 - Quantitative measurement of TSH level in non-spiked plasma samples

The goal of the experiment is to establish a linear curve for TSH in non-spiked plasma samples with different TSH concentration.

Materials

Materials used in this example:

- Screen-printed gold electrodes (Dropsens C220BT)
- Milli-Q water
- 5 mM HEPES buffer pH 7
- 0.3 mM Peptide (SEQ ID 35 - also called P4) in 5 mM HEPES pH 7 (peptide)
- 2 mM potassium ferri/ferrocyanide containing 200 mM KCl
- 1 mM Mercaptohexanol (MCH) in Milli-Q water;
- Plasma sample with TSH content 0.05, 1.4, 4.5, and 8.3 mIU/L

Electrode cleaning

Cleaning was performed by incubating the electrode in 50 mM KOH/25% $H_2O_2$ (80 $\mu$l) for about 20 min followed by 1 round of Cyclic Voltammetry (CV) in 50 mM KOH for one cycle between -0,2 V to -1,2 V at 50 mV/s. The electrode was flushed with ultra-pure water (Milli-Q water) and $N_2$-dried after both KOH/$H_2O_2$ incubation and CV cycling in KOH.

Peptide immobilization

0.3 mM peptide (SEQ ID 35) solution in 5 mM HEPES buffer, pH 7, was prepared by diluting down from 5 mM stock solution in Milli-Q water. Then, 20 $\mu$l of this solution was placed on the working part of the electrode only, by ensuring the working electrode is covered entirely and uniformly without overflowing to the reference or the counter side. The electrodes were placed in a Petri dish that contained water-soaked tissue paper under them and kept in a fridge overnight. At the end of incubation, electrodes were rinsed with Milli-Q water (with a gentle spray above the electrodes, not directly on them) followed by a gentle nitrogen blowing to a slightly dry state.

MCH incubation

First, 5 mM MCH in water was prepared by mixing 3.5 $\mu$l of MCH stock solution with 5 ml Milli-Q water. Then, further dilution to 1 mM was prepared in Milli-Q water. On the rinsed and slightly dry electrode, 20 $\mu$l of MCH in Milli-Q (freshly prepared) was placed and let incubate for 30 min in a Petri dish with wet tissues under the electrodes, and were incubated for 1 hour. Then, the electrodes were gently rinsed by flushing Milli-Q above the sensor part. Then, the electrodes were transferred into a tube containing Milli-Q water and placed on a shaker for 15 min at 700 rpm. After shaking, they were removed and gently rinsed with Milli-Q and nitrogen dried.

TSH and control sample incubation

The rinsed and slightly dried electrode was incubated with 20 $\mu$l of plasma samples containing different concentration of TSH. Plasma samples with TSH content of 0.05, 1.4, 4.5 and 8.3 mIU/L were used to draw the calibration plot. The sample was incubated for 60 min while kept on a shaker at 500 rpm followed by gentle rinsing with Milli-Q water and $N_2$-drying.

EIS measurement

80 $\mu$l of 2 mM ferri/ferro solution containing 200 mM KCl was placed on the electrode and the spectra was recorded.

EIS parameters

The following EIS parameters were used for the measurements of this example:

- Open Circuit Potential (OCP)
- First applied frequency: 1 MHz
- Last applied frequency: 0.1 Hz
- AC amplitude: 5 mV

All measurements were performed on a six-channel electrode connector arrays.

Testing conditions

All the experiments were conducted in triplicates unless specified.

Results

A linear relationship was established between Rct (charge transfer resistance) readings and TSH content in raw plasma samples between 0.05 and 8.3 mIU/L (normal human TSH level is 0.5-5mIU). The results are further shown in figure 2, and values are also given in table 1 below.

Table 1 - Rct values that are corresponding with the samples TSH level.

| [TSH] (mIU/L) | Mean $R_{ct}$ (k$\Omega$) | SD | %RSD |
|---|---|---|---|
| **Run 1** | | | |
| 0.05 **mIU** | 0.98 | 0.09 | 9.32 |
| 1.4 **mIU** | 1.21 | 0.10 | 8.38 |
| 4.5 mIll | 1.25 | 0.10 | 7.91 |
| 8.3 **mIU** | 1.51 | 0.15 | 9.62 |
| **Run 2** | | | |
| 1.4 **mIU** | 0.37 | 0.08 | 20.90 |
| 3.5 **mIU** | 0.45 | 0.06 | 14.48 |
| 5.2 **mIU** | 0.53 | 0.11 | 21.17 |
| 8.3 **mIU** | 0,60 | 0.12 | 20.49 |
| **Run 3** | | | |
| 2.1 **mIU** | 1.35 | 0.01 | 0.37 |
| 3.5 **mIU** | 1.71 | 0.22 | 13.06 |
| 5.2 **mIU** | 2.00 | 0.35 | 17.66 |
| 9.6 **mIU** | 2.59 | 0.08 | 2.92 |
| **Table 1** | | | |

Table 1 summarizes the sample based measurements data (mean Rct values of triplicate measurement) with corresponding standard deviation (SD) and %RSD calculation.

Rerun

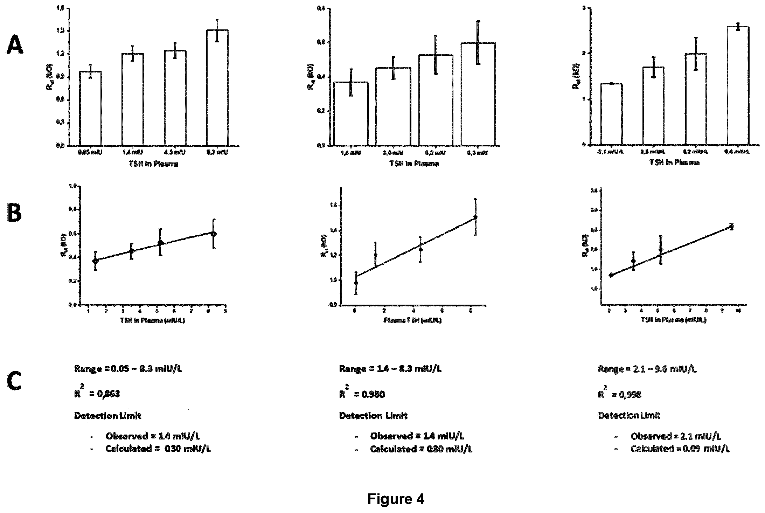

The experiment was run two additional times. The results are shown in figure 4. Run 2 and 3 were done according to the above procedure, only difference being that plasma samples with different TSH content were used.

Conclusion

Plasma samples of different TSH content were analysed in three independent runs. Sample concentrations were selected to include the physiological range in normal humans. The Rct values obtained from EIS fitting followed a very good correlation to the concentration of TSH. This demonstrates the excellent performance of the method to detect TSH in complex plasma sample.

Example 2 - EIS signal correlation between spiked plasma samples and native plasma samples

The goal of the experiment is to determine the ability of the peptide (SEQ ID 35) to detect TSH in spiked plasma samples and to observe the correlation between native and spiked plasma samples.

Materials

Materials used in this example:

- Screen-printed gold electrodes (DropSens C220BT), 24 in total.
- Milli-Q water
- KOH solution (50 mM):

    A 500 mM KOH (potassium hydroxide) stock solution is made by adding 2.805g to 100 mL Milli-Q water
    Make a 50 mL 50 mM KOH solution by mixing 5 mL of KOH 500 mM with 45 mL of distilled water

- Ferri/ferrocyanide:

    $Fe(CN)_6^{-3/-4}$ 2 mM (containing 200 mM KCl)
    Use 500 ml DI water
    Add 0.32924 g Potassium ferricyanide ($K_3Fe(CN)_6$, MW=329.24)
    Add 0.42239 g Potassium ferrocyanide ($K_4Fe(CN)_6$, MW= 422.39)
    Add 7.45 g Potassium chloride (KCL)

- MCH:
    Prepare 5mM MCH solution in Milli-Q water by diluting 3.5 uL MCH in 5 mL Milli-Q water. Prepare 1mM MCH solution by diluting 200 uL 5mM MCH solution in 800 uL Milli-Q water.
- HEPES 5 mM, pH 7.0
- TSH and 0.05 mIU/L TSH plasma samples
- Anti-TSH peptide (SEQ ID 35) (0.3 mM)

Procedure

12 C220BT electrodes were used in order to determine the biosensing performance of peptide (SEQ ID 35) in patient samples. 0.05 mIU/L plasma was spiked with TSH to three final concentrations of 1.2, 8.3 and 49.9 mIU/L TSH. These concentrations were chosen in order to compare TSH spiked plasma samples EIS response with native plasma samples.

Each step described below should be performed for each individual chip used. The electrodes are kept in the connector array until the final EIS measurement is performed. The electrodes were gently dried using air after each rinsing step. Each incubation step is performed in a closed container to prevent evaporation.

Cleaning of electrodes

    1. Electrodes were cleaned with $KOH/H_2O_2$ solution by pipetting 50 μL on the electrode surface and reacting for 10 minutes.

2. The electrodes were rinsed with Milli-Q water.

3. The chips were connected with the potentiostat (PGSTAT204).

4. Add 50 mM KOH (80 $\mu$L) to the chip surface so that it covers all three electrodes.

5. Perform one CV cycle in 50 mM KOH solution, from -0.2 V to -1.2 V at a scan rate of 50 mV.

6. Rinse the electrodes by spraying the surface of the electrode with Milli-Q water and dry using air in the fume hood.

Functionalization with peptide (SEQ ID 35)

1. Cleaned chips were incubated with 15 $\mu$L, 0.3 mM/ml P4 (P4 is peptide with SEQ ID 35) suspension for 2 hours at room temp.

2. After the incubation period, the electrodes were rinsed with 500 $\mu$L 5mM, pH 7 HEPES, followed by suspending 80 $\mu$L HEPES and pipetting 8 times and a last rinsing step using 500 $\mu$L HEPES.

Blocking with MCH

1. The peptide (SEQ ID 35) modified electrodes were incubated with 15 $\mu$L, 1mM MCH solution for 1h at room temp.

2. After the incubation period, the electrodes were rinsed with 500 $\mu$L HEPES.

3. Two consecutive suspensions were also used to further remove weakly bound MCH placing 80 $\mu$L HEPES on the electrode surface and pipetting 8-10 times.

4. One last rinsing step is performed with 500 $\mu$L HEPES.

TSH incubation

1. The peptide (SEQ ID 35) modified and MCH blocked electrodes were incubated with 15 $\mu$L spiked plasma samples (done in triplicates).

2. The control electrodes were incubated with the same volume of 0.05 mIU/L plasma.

3. The electrodes were rinsed twice with 500 $\mu$L HEPES, followed by suspending 80 $\mu$L HEPES and pipetting 8 times.

4. Additional rinsing step using 500 $\mu$L HEPES, two times.

5. EIS is performed to observe the changes in Rct caused by the capture of TSH or FSH to the surface of the electrode by the peptide (SEQ ID 35).

EIS measurements

1. An 80 $\mu$L droplet of ferri/ferrocyanide is placed on the electrodes. Make sure to cover all 3 electrodes (WE, CE, RE).

2. Perform EIS with parameters indicated below, using triplicates and control electrodes:

- EIS parameters
- DC bias; 0 vs OCP
- First applied frequency: 100 KHz
- Last applied frequency: 0.1 Hz
- AC amplitude: 5 mV

Testing conditions

EIS measurements are to be carried out using the small AutoLab potentiostat using a 6 channel connector array.

Results

The results (figure 3) show a relationship between TSH concentration and measured Rct values. This indicates that different TSH concentrations can be successfully identified using EIS. The linear regression fit shows a good correlation between all three concentrations, with an $R^2$=0.84. All measured data including RSD and coefficient of variation are shown in table 2.

| Sample # | 49.9 mIU/L | 8.3 mIU/L | 1.4 mIU/L | 0.05 mIU/L |
|---|---|---|---|---|
| 1 | 1430 | 1290 | 1740 | N/A |
| 2 | 1780 | 1819 | 1500 | 1310 |
| 3 | 2010 | 1830 | 1410 | 1230 |
| Mean | 1740 | 1646 | 1550 | 1270 |
| SD | 168 | 178 | 98 | 40 |
| RSE% | 9.7 | 10.8 | 6.4 | 3.1 |
| **Table 2** | | | | |

A strong correlation was observed between spiked plasma samples and native plasma samples. The result of both experiments is overlaid and shown in figure 3. Both experiments follow the same linear trend and further suggest that peptide (SEQ ID 35) modified electrodes can detect TSH in native and spiked plasma samples.

Conclusion

Human plasma samples (0.05 mIU/L) were artificially spiked at corresponding TSH concentrations of the natural plasma samples in order to determine the correlation of known naturally occurring TSH vs the controlled laboratory scenario. A similar trend and correlation were observed between natural and artificially spiked plasma. This suggests that the system as disclosed herein is reproducible and can quantitatively measure TSH in complex media.

Example 3 - Native TSH plasma using direct peptide attachment and MCH blocking

The goal of this experiment is to observe if direct P4 attachment with decreased MCH incubation time (30 min) offers better results overall, when testing in native plasma samples.

Materials

Materials used in this example:

- Screen-printed gold electrodes (DropSens C220BT) 24 in total
- Milli-Q water
- KOH solution (50 mM) - see example 2
- Ferri/ferrocyanide - see example 2
- 1 mM MCH - see example 2
- HEPES 5 mM, pH 7.0
- TSH plasma samples
- Anti-TSH peptide (SEQ ID 35) (0.3 mM)

Procedure

Measurements were performed on peptide (SEQ ID 35) modified C220BT electrodes using 5 plasma samples with different native TSH concentrations. Each step described below should be performed for each individual chip used. The electrodes are kept in the connector array until the final EIS measurement is performed. The electrodes were gently dried using air after each rinsing step. Each incubation step is performed in a closed container to prevent evaporation.

Cleaning of electrodes

1. Electrodes were cleaned with KOH/$H_2O_2$ solution by pipetting 50 $\mu$L on the electrode surface and reacting for 10 minutes

2. The electrodes were rinsed with Milli-Q water.

3. The chips were connected to the potentiostat (PGSTAT204).

4. Add 50 mM KOH (80 µL) to the chip surface so that it covers all three electrodes.

5. Perform one CV cycle in 50 mM KOH solution, from -0.2 V to -1.2 V at a scan rate of 50 mV

6. Rinse the electrodes by spraying the surface of the electrode with Milli-Q water and dry using air in the fume hood.

Functionalization with peptide (SEQ ID 35)

1. Cleaned chips were incubated with 15 µL, 0.3 mM/ml peptide (SEQ ID 35) suspension for 2 hours.

2. After the incubation period, the electrodes were rinsed with 5 mL of Milli-Q water from the squeeze bottle and dried using nitrogen gas.

Blocking with MCH

1. The peptide (SEQ ID 35) modified electrodes were incubated with 15 µL 1mM MCH solution for 30 min at room temperature.

2. After MCH incubation is over, the electrodes were rinsed once with 500 µL 5 mM HEPES after which 80 µL 5mM HEPES were suspended on the electrode. The weakly attached MCH molecules are removed by pipetting the suspension 20 times.

3. One last rinsing step was performed with Milli-Q from the squeeze bottle.

4. The electrodes were dried using nitrogen gas.

TSH incubation

1. The peptide (SEQ ID 35) modified and MCH blocked electrodes were incubated with 15 µL plasma samples at different TSH concentrations for 30 minutes.

2. The incubation uses a time offset of t = 5min (enough to rinse the electrode, dry and measure until the incubation time for the next electrode is up)

3. The first electrode is incubated with TSH plasma while the other electrodes are kept wet with 80 uL of 5mM HEPES.

4. After 5 minutes the next electrode is dried to remove the HEPES and incubated with TSH plasma. This is repeated for all electrodes.

5. Once 30 min passes (TSH incubation duration), the first chip is rinsed with Milli-Q water from the squeeze bottle, dried using nitrogen gas and measured using EIS. Once the EIS measurement is finished the next electrode should have reached 30 minutes and the rinsing and drying and EIS measurement is repeated for each individual electrode until the experiment is finished.

EIS measurements

1. An 80 µL droplet of ferri/ferrocyanide is placed on the electrodes. Make sure to cover all 3 electrodes (WE, CE, RE).

2. Perform EIS with parameters indicated below, using triplicates and control electrodes:

- EIS parameters
- DC bias; 0 vs OCP
- First applied frequency: 100 KHz
- Last applied frequency: 0.1 Hz
- AC amplitude: 5 mV

Testing conditions

EIS measurements are to be carried out using the small AutoLab potentiostat using electrode connectors arrays without shaking during the incubation step.

Results

The results are shown in figure 5. The results are further shown in table 7 below. The Rct values are obtained by fitting the raw data using a python script.

|  | 9.6mlU | 5.2mlU | 3.5mlU | 2.1mlU | 0.14mlU |
|---|---|---|---|---|---|
| Mean | 2590 | 2275 | 1710 | 1345 | 2005 |
| SD | 131 | 530 | 316 | 7 | 21 |
| %RSD | 5.1 | 23.3 | 18.5 | 0.5 | 1.1 |
| **Table 3** | | | | | |

From the plotted Rct values it can be observed that there is a correlation in the range of 2.1 to 9.6 mIU/L in native plasma samples. In this case the lowest concentration, 0.14 mIU/L was identified as an outlier and was not used in the regression analysis. The Rct values obtained in the range of 2.1 to 9.6 mIU/L were analyzed to construct a standard curve (not shown). It was observed that there was a linear trend in this concentration range. The correlation between all the concentration is strong with an $R^2$ value of 0.97. The SD was used to construct the error bars shown in figure 5 and are also presented in table 7. A limit of detection (LOD) of 0.3 mIU/L was calculated based on the following formula:

$$LOD = \frac{3xSD}{m}$$

Where SD is the standard deviation of the slope and m is the slope of the calibration curve.

Conclusion

The results show that the MCH incubation duration can be reduced to 30 minutes. LOD was determined by the above-mentioned calculation using the standard curve obtained between 2.1 and 9.6 mIU/L and 3.2 and 9.6 mIU/L.

Example 4 - Precision and accuracy tests of our TSH measurement

Materials

Materials used in this example:

- Flex Medical Electrodes (24 in total)
- Milli-Q water
- 5 mM HEPES buffer, pH 7
- 0.3 mM peptide (SEQ ID 35) in 5 mM HEPES, pH 7
- 2 mM ferri/ferrocyanide containing 200 mM KCI
- 1 mM Mercaptohexanol (MCH) in Milli-Q water
- Plasma sample with 5.0 mIU/L
- HEPES with TSH content 7.0 mIU/L

Electrode cleaning

Cleaning was performed by CV in 50 mM KOH for one cycle between -0.2 V to -1.2 V at 50 mV/s. The electrode was flushed with Milli-Q water and $N_2$-dried after CV cycling.

Peptide immobilization

0.3 mM peptide (SEQ ID 35) solution in 5 mM HEPES buffer, pH 7, was prepared by diluting down from 5 mM stock solution in Milli-Q water. Then, 15 $\mu$l of this solution was placed on the working electrode only, by ensuring it is covered entirely and uniformly without overflowing to the reference or the counter side. The electrodes were placed in a Petri dish that contained water-soaked tissue paper under them and kept in a fridge overnight. At the end of incubation, electrodes were rinsed with Milli-Q water (with a gentle spray above the electrodes, not directly on them) followed by gentle nitrogen blowing to a slightly dry state.

MCH incubation

First, 5 mM MCH in water was prepared by mixing 3.5 $\mu$l of MCH stock solution with 5 ml Milli-Q water. Then, further dilution to 1 mM was prepared in Milli-Q water. On the rinsed and slightly dry electrode, 15 $\mu$l of 1mM MCH in Milli-Q water (freshly prepared as described above) was placed and left to incubate for 30 min in a Petri dish with wet tissues under the electrodes. Then, the electrodes were gently rinsed by flushing Milli-Q above the sensor part. Then, the electrodes were transferred into a tube containing Milli-Q water and placed on a shaker for 15 min at 700 rpm. After shaking, they were removed and gently rinsed with Milli-Q water and nitrogen dried.

TSH incubation

The rinsed and slightly dry electrode was incubated with 15 $\mu$l of plasma samples. In this study, plasma sample with TSH level of 5.0 mIU/L and 5 mM HEPES with 7.0 mIU/L TSH were used, i.e. in this experiment, parallel test was performed one in native plasma containing 5.0 mIU TSH whereas the other was a buffer medium with 7.0 mIU TSH. These were incubated for 60 min while kept on a shaker at 500 rpm followed by gentle rinsing with Milli-Q water and $N_2$-drying. Then, the electrodes were kept in 5 mM HEPES, pH 7, until EIS measurement were to be commenced. In both cases, electrodes were quickly blown with nitrogen just before the EIS measurement.

EIS measurement

80 $\mu$l of 2 mM ferri/ferro solution containing 200 mM KCl was placed on the electrode and the spectra was recorded.

EIS parameters

- OCP
- First applied frequency: 2e5 Hz
- Last applied frequency: 0.1 Hz
- AC amplitude: 5 mV

All measurements were performed on a single channel using electrode connecting arrays.

Testing conditions

Each sample was analysed on twelve sensors

Results

The results are shown in figure 6 shows the repeatability of the method evaluated by taking twelve independent measurements in 5.0 mIU/L patient plasma sample. The calculated relative deviation standard (RSD) was found to be 12.2%. Similarly, a repeatability test was performed in 7.0 mIU/L TSH spiked 5 mM HEPES buffer, pH 7. The RSD value obtained from twelve repetitive tests was 12.0% which is comparable to the results obtained in patient plasma.

Conclusion

The precision of the method was evaluated by taking twelve repetitive measurements in 5.0 mIU/L TSH in human plasma and 7.0 mIU/L TSH in spiked buffer solution. The calculated RSD values from the charge transfer resistance was 12.2% and 12.0%, respectively, in plasma and buffer. This indicates that the repeatability is not affected by the sample matrix. Moreover, the calculated precision is good considering the possibility of improvement in handling steps during sensor preparation and measurement.

Example 5 - Interference and specificity tests of our TSH measurement

The goal of the experiment is to show the specificity towards TSH.

Materials

Materials used in this example:

- Screen-printed gold electrodes (DropSens C220BT) 24 in total.
- Milli-Q water
- KOH solution (50 mM) - see example 2
- Ferri/ferrocyanide -see example 2
- dithiobis(succinimidyl propionate) (DSP)
  Prepare 10 mM DSP solution in dimethyl sulfoxide (DMSO) by dissolving 10 mg DSP powder in 5 mL DMSO.
- 1 mM MCH - see example 2
- HEPES 5 mM, pH 7.0
- 50 ng/mL TSH in 5 mM HEPES, pH 7
- 50 ng/mL FSH in 5 mM HEPES, pH 7
- 1;1 TSH/FSH (50 ng/mL) in 5mM HEPES, pH 7
- Anti-TSH peptide (SEQ ID 35) (0.3 mM)

Procedure

12 C220BT electrodes were used to determine the specificity of peptide (SEQ ID 35) towards TSH. A total set of 3 experiments (12 electrodes each) was used in order to determine specificity and to possibly optimize the sensor. Each step described below should be performed for each individual chip used. The electrodes are kept in the connectors until the final EIS measurement is performed. The electrodes were gently dried using air after each rinsing step. Each incubation step is performed in a closed container to prevent evaporation.

Cleaning of electrodes

1. Electrodes were cleaned with $KOH/H_2O_2$ solution by pipetting 50 $\mu$L on the electrode surface and reacting for 10 minutes.
2. The electrodes were rinsed with Milli-Q water.
3. The chips are connected to the potentiostat (PGSTAT204).
4. Add 50 mM KOH (80 $\mu$L) to the chip surface so that it covers all three electrodes.
5. Perform one CV cycle in 50 mM KOH solution, from -0.2 V to -1.2 V at a scan rate of 50 mV
6. Rinse the electrodes by spraying the surface of the electrode with Milli-Q water and dry using air in the fume hood.

Electrode functionalization with DSP

1. The previously cleaned electrodes were incubated with 40 $\mu$L 10mM DSP for 1.5 hours in a closed container to prevent evaporation.
2. It is very important to use freshly prepared DSP solutions since DSP contains a reactive NHS-ester which hydrolyzes in solution. DSP powder is hygroscopic and should be equilibrated at room temperature before use. Keep at room temperature for as little time as possible.
3. After incubation, the electrodes were rinsed by suspending 80 $\mu$L DMSO on each chip and pipetting 8 times after which the electrodes are rinsed with 5 mL of Milli-Q water directly from the squeeze bottle to remove any unbound DSP.

Functionalization with peptide (SEQ ID 35)

1. Cleaned chips were incubated with 15 $\mu$L 0.3 mM/mL or 15 $\mu$L 0.15 mM/ peptide (SEQ ID 35) suspension for 2 hours at room temp.
2. After the incubation period, the electrodes were rinsed with 500 $\mu$L HEPES, followed by suspending 80 $\mu$L HEPES and pipetting 8 times and a last rinsing step using 500 $\mu$L HEPES.

Blocking with MCH

1. The peptide (SEQ ID 35) modified electrodes were incubated with 15 $\mu$L 1mM MCH solution for 30 min at room temp.
2. After the incubation period, the electrodes were rinsed with 500 $\mu$L HEPES. Two consecutive suspensions were also used to further remove weakly bound MCH placing 80 $\mu$L HEPES on the electrode surface and pipetting 8-10 times. One last rinsing step is performed with 500 $\mu$L HEPES.

TSH incubation

1. The peptide (SEQ ID 35) modified and MCH blocked electrodes were incubated with 15 $\mu$L 50 ng/mL TSH or 15 uL 50 ng/mL FSH or 15 uL mixed, 50 ng TSH/FSH for 1 hour while shaking at 400 RPM
2. The control electrodes were incubated with the same volume of 5 mM pH 7 HEPES buffer pH 7.
3. The electrodes were rinsed with twice 500 $\mu$L HEPES, followed by suspending 80 $\mu$L HEPES and pipetting 8 times and a last rinsing step using 500 $\mu$L HEPES, two times.

EIS measurements

EIS is performed to observe the changes in Rct caused by the capture of TSH or FSH to the surface by peptide (SEQ ID 35).

1. Perform EIS with parameters indicated below, using triplicates and control electrodes:

- EIS parameters
- DC bias; 0 vs OCP
- First applied frequency: 100 KHz
- Last applied frequency: 0.1 Hz
- AC amplitude: 5 mV

Testing conditions

EIS measurements are to be carried out using the small AutoLab potentiostat using a 6-channel electrode connector array.

Results

The interference study results are shown in figure 7. The changes of Rct (the charge transfer resistant) values are observed by TSH (50ng/mL) binding to peptide (SEQ ID 35), while FSH (50ng/ml) alone does not modify the charge transfer signal, thus peptide (SEQ ID 35) does not bind FSH. When a mixture of TSH and FSH (50ng/mL from each) are used the signal increases, however, not as much as TSH alone. There could be different reasons such as the electrode inhomogeneity or the most probably the assay reached its limitation and TSH binding is hindered by protein saturation. This will be addressed and improved by optimizing the blocking and passivation of the electrode in further studies.

Conclusion

TSH (50 ng/mL) incubated electrodes show a high response during EIS measurement while FSH (50 ng/mL) incubated electrodes show the same response as the negative control electrodes. The response of TSH and FSH (1:1 ratio; 50 ng/mL) mixed samples show a similar response as the TSH only electrodes. The data suggests that peptide modified electrodes are sensitive and selective towards TSH.

**Claims**

1. An electrochemical biosensor system for measuring concentrations of an analyte in a fluid, the electrochemical biosensor system comprises an electrochemical sensor, the electrochemical sensor comprising an electrode system comprising a working electrode, a counter electrode, and a reference electrode;

   wherein the working electrode comprises a first surface having a predefined electron transfer property, the first surface being in contact with the fluid when the electrochemical biosensor system is in use;
   wherein a binding agent is attached to the first surface, the binding agent being configured for specifically binding to the analyte to form a binding agent-analyte complex, wherein formation of the binding agent-analyte complex alters the predefined electron transfer property of the first surface of the working electrode, thereby providing a change in an electrochemical response at the first surface of the working electrode proportional to the number of binding agent-analyte complexes formed; and
   wherein the analyte is thyroid-stimulating hormone (TSH) and wherein the binding agent is one or more peptides comprising a binding motif for the analyte having sequences deriving from or similar to thyroid-stimulating hormone receptor (TSHR).

2. The electrochemical biosensor system according to claim 1, wherein the working electrode is a gold electrode.

3. The electrochemical biosensor system according to any of the preceding claims, wherein the working electrode has an electrical impedance, wherein the electrical impedance changes in response to the formation of the binding agent-analyte complex.

4. The electrochemical biosensor system according to any of the preceding claims, wherein the binding agent is immobilized on the first surface of the working electrode.

5. The electrochemical biosensor system according to any of the preceding claims, wherein the electrochemical biosensor system further comprises a computing unit cable of receiving a first input from the electrode system, the first input corresponding to the change in the electrochemical response at the first surface of the working electrode proportional to the number of binding agent-analyte complexes formed, and wherein the computing unit configured for transmitting data to an end user based on the received first input.

6. The electrochemical biosensor system according to any of the preceding claims, wherein the binding agent is covalently bound to the first surface of the working electrode.

7. The electrochemical biosensor system according to any of the preceding claims, wherein the electrochemical biosensor system further comprises a potentiostat and a microprocessor, wherein the working electrode, the counter electrode, and the reference electrode are connected to the potentiostat, and wherein the potentiostat is connected to the microprocessor.

8. The electrochemical biosensor system according to any of the preceding claims, wherein the microprocessor is adapted for receiving data from the electrochemical sensor and transmitting data to an end user.

9. The electrochemical biosensor system according to any of the preceding claims, wherein the electrochemical biosensor system comprises two parts detachably connected, the two parts including:

   a disposable part comprising at least the electrode system comprising the working electrode, the counter electrode, and the reference electrode; and
   a reusable part comprising a potentiostat and a microprocessor;

wherein the working electrode, the counter electrode, and the reference electrode are connected to the potentiostat, and wherein the potentiostat is connected to the microprocessor.

10. The electrochemical biosensor system according to any of the preceding claims, wherein the electrochemical biosensor system further comprises a sample handling unit wherein the sample handling unit comprises an integrated blood to plasma separation unit.

11. The electrochemical biosensor system according to any of the preceding claims, wherein the electrochemical biosensor system further comprises a flow control, wherein the flow control comprises an automatic reagent release system configured to release plasma and one or more reagents to the electrode system, and wherein at least one of the one or more reagents are ferrocyanide.

12. The electrochemical biosensor system according to any of the preceding claims, wherein the electrochemical biosensor system further comprises a waste collection unit.

13. The electrochemical biosensor system according to any of the preceding claims, wherein the electrochemical biosensor system further comprises an additional sensor system wherein the additional sensor system is configured to ensure a predefined quality of a readout provided by the electrode system.

14. Use of electrochemical biosensor system according to any of claims 1-13 for measuring a concentration of an analyte in a fluid, wherein the analyte is thyroid-stimulating hormone (TSH).

15. A method of measuring a concentration of an analyte in a fluid wherein the analyte is thyroid-stimulating hormone (TSH), the method comprising:

a. providing an electrochemical biosensor system according to any of claims 1-13;
b. providing a fluid sample;
c. contacting the working electrode with the fluid sample under conditions that allow the formation of the binding agent-analyte complexes for a period of time sufficient to allow the formation of the binding agent-analyte complexes.

**Figure 1**

Gold electrode

1. Clean gold electrode

2. Surface functionalization with peptide

3. Blocking remaining surface

4. Incubation with the analyte (TSH)

5. Electrochemical Impedance (EIS) measurements

Peptide    Surface blocker    TSH    Probe reagent solution

Figure 2

Figure 3

Figure 4

**Figure 5**

**Figure 6**

**Figure 7**

Europäisches
Patentamt
European
Patent Office
Office européen
des brevets

# EUROPEAN SEARCH REPORT

**Application Number**

EP 22 18 4953

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 112 881 492 A (CHANGSHA XINLI ZHIHE TECH CO LTD) 1 June 2021 (2021-06-01) <br> * figures 1-8 * <br> * paragraph [0009] – paragraph [0227] * | 1-15 | INV. <br> G01N27/327 <br> G01N33/543 <br> G01N33/78 |
| X | US 2021/132050 A1 (CILLI EDUARDO MAFFUD [BR] ET AL) 6 May 2021 (2021-05-06) <br> * figures 1-3 * <br> * paragraph [0008] – paragraph [0120] * | 1-15 | |
| X | US 2015/247816 A1 (BHANSALI SHEKHAR [US] ET AL) 3 September 2015 (2015-09-03) <br> * figures 1-34 * <br> * paragraph [0005] – paragraph [0163] * | 1-15 | |
| A | BENDO LUANA ET AL: "Nanostructured Sensors Containing Immobilized Nuclear Receptors for Thyroid Hormone Detection", JOURNAL OF BIOMEDICAL NANOTECHNOLOGY, vol. 10, no. 5, 1 May 2014 (2014-05-01), pages 744-750, XP093010177, US <br> ISSN: 1550-7033, DOI: 10.1166/jbn.2014.1774 <br> Retrieved from the Internet: URL:http://dx.doi.org/10.1166/jbn.2014.1774> <br> * page 744 * | 1-15 | TECHNICAL FIELDS SEARCHED (IPC) <br><br> G01N |

The present search report has been drawn up for all claims

| Place of search | Date of completion of the search | Examiner |
|---|---|---|
| Munich | 23 December 2022 | Colasanti, Katharina |

EPO FORM 1503 03.82 (P04C01)

## ANNEX TO THE EUROPEAN SEARCH REPORT
## ON EUROPEAN PATENT APPLICATION NO.

**EP 22 18 4953**

**23-12-2022**

| Patent document cited in search report | | | Publication date | Patent family member(s) | | Publication date |
|---|---|---|---|---|---|---|
| CN 112881492 | A | | 01-06-2021 | NONE | | |
| US 2021132050 | A1 | | 06-05-2021 | BR 112020014894 | A2 | 08-12-2020 |
| | | | | EP 3743715 | A1 | 02-12-2020 |
| | | | | US 2021132050 | A1 | 06-05-2021 |
| | | | | WO 2019145706 | A1 | 01-08-2019 |
| US 2015247816 | A1 | | 03-09-2015 | NONE | | |